(19) Europäisches Patentamt

European Patent Office

Office européen des brevets

(11) Veröffentlichungsnummer: **0 271 438 B1**

(12) **EUROPÄISCHE PATENTSCHRIFT**

(45) Veröffentlichungstag der Patentschrift: **29.04.92**

(51) Int. Cl.⁵: **A61K 9/22**

(21) Anmeldenummer: **87810646.7**

(22) Anmeldetag: **09.11.87**

54 **Orales osmotisches System für Metoprolol mit verbesserten Formulierungseigenschaften.**

(30) Priorität: **14.11.86 US 930828**

(43) Veröffentlichungstag der Anmeldung:
**15.06.88 Patentblatt 88/24**

(45) Bekanntmachung des Hinweises auf die
Patenterteilung:
**29.04.92 Patentblatt 92/18**

(84) Benannte Vertragsstaaten:
**AT BE CH DE ES FR GB GR IT LI LU NL SE**

(56) Entgegenhaltungen:
**GB-A- 2 135 880**
**US-A- 3 916 899**

(73) Patentinhaber: **CIBA-GEIGY AG**
**Klybeckstrasse 141**
**CH-4002 Basel(CH)**

(72) Erfinder: **Lee, Shih-Wei**
**278 Brandywine Drive**
**Orangeburg New York 10962(US)**

**Beschreibung**

Die Erfindung betrifft ein orales osmotisches System für mässig wasserlösliche Metoprololsalze, welches verbesserte Formulierungseigenschaften besitzt, und die therapeutische Verwendung dieses Systems bei der Bekämpfung von Krankheiten und Zuständen, in denen der Einsatz von Beta-Adrenalinrezeptorblockern indiziert ist.

Orale osmotische Systeme für die therapeutische Verabreichung von Wirkstoffen sind seit langem bekannt. Diese Systeme dienen zur kontrollierten und verzögerten Abgabe von Wirkstoffen über einen verlängerten Verabreichungszeitraum hinweg und werden z.B. in den U.S. Patentschriften 3.845,770; 3,916,899 sowie 4,016,880 beschrieben.

Ein orales orsmotisches System für die therapeutische Verabreichung des Metoprololfumarat-Salzes mit einer semipermeablen Beschichtung aus Celluloseacetat und das in viv-Abgabeverhalten dieser Darreichungsform ist von F. Theeuwes et al. in drei Uebersichtsartikeln in Br.J.Clin.Pharm. Vol. 19, 1985 auf den Seiten 69-76S, 213-218S und 219-224S beschrieben worden.

Solche Darreichungsformen mit einem mässig wasserlöslichen Metoprololsalz wie Metoprololfumarat (1:1) im Kern des therapeutischen Systems sind bei der grosstechnischen Herstellung mit hohen Ansprüchen an die Einheitlichkeit der Zusammensetzung und mechanische Beanspruchung technisch nachteilig: Aufgrund der hohen Sprödigkeit des Metoprololsalzes, sind komprimierte Tablettenkerne, welche ausschliesslich aus Wirkstoff oder Wirkstoff mit geringen Zusätzen wie Poly-N-vinylpyrrolidon bestehen, nicht bruchfest genug, um in den üblichen Beschichtungsverfahren wie Wursters Luftverwirbelungsverfahren verwendet zu werden.

Bei Verwendung grösserer Mengen an Hilfsstoffen ist ausserdem eine ungünstige Beeinflussung des Abgabeverhaltens durch Verminderung der Abgabemenge zu erwarten.

Der vorliegenden Erfindung liegt die Aufgabe zugrunde, ein technisch vorteilhaftes orales osmotisches System für ein mässig wasserlösliches und pharmazeutisch annehmbares Metoprololsalz bereitzustellen.

Es wurde überraschenderweise gefunden, dass das bekannte und nachteilige Bruchverhalten von Tablettenkernen sich verhindern lässt, wenn man der Formulierungsmasse des Kerns eine Gesamtmenge von 7,5-15 % Poly-N-vinylpyrrolidon bezogen auf das Gesamtgewicht des Tablettenkerns bei Verwendung eines mässig wasserlöslichen und pharmazeutisch annehmbaren Metoprololsalzes hinzusetzt.

Das erfindungsgemässe orale osmotische System besteht aus folgenden Bestandteilen

a) einer äusseren Hülle aus einem für Wasser durchlässigen und für die Komponenten des wirkstoffhaltigen Kerns undurchlässigen semipermeablem Material,

b) einem Kern mit einem zur Erzeugung von osmotischem Druck ausreichend wasserlöslichen Gemisch aus 7,5 bis 15 Gew-% Poly-N-vinylpyrrolidon, ca. 5 Gew-% eines bei der Herstellung von festen Darreichungsformen üblichen Gleit- oder Schmiermittels und 80 bis 92,5 % eines mässig wasserlöslichen und pharmazeutisch annehmbaren Metoprololsalzes und

c) einer Passage durch die Hülle a) für den Transport der im Kern enthaltenen Bestandteile in die umgebende wässrige Körperflüssigkeit.

Das erfindungsgemässe orale osmotische System für die Verabreichung eines Metoprololsalzes eignet sich für die Verwendung in Heilverfahren zur Behandlung von Krankheiten oder Zuständen, welche für die Verwendung von $Beta_1$-Adrenalinrezeptorblockern (Betablocker) indiziert sind. Bevorzugt sind die Indikationen, welche für Metoprolol selbst und seine pharmazeutisch annehmbaren Salze bekannt sind, insbesondere Bluthochdruck, Angina Pectoris, Herzrhythmusstörungen oder bei der Nachsorge von Patienten mit Myokardinfarkt.

Beim Handelspräparat Metoprololtartrat ist das Abgabeverhalten der Darreichungsform durch sofortige Abgabe des Wirkstoffs gekennzeichnet. Es fehlt ein geschwindigkeitskontrolliertes, kontinuierliches Abgabeverhalten. Bei erforderlicher Mehrfachdosierung erzeugen solche Formulierungen grosse Schwankungen des Blutplasmaspiegels zwischen hohen und niedrigen Konzentrationen des Wirkstoffs unter Verminderung der Effektivität der Betablockade. Häufige Zufuhr des Wirkstoffs kann zwar das Absinken des Blutplasmaspiegels unter das therapeutische Niveau verhindern, stellt aber insgesamt eine Belastung des Organismus dar. Ausserdem besteht das Risiko der unbeabsichtigten nicht regelmässigen Einnahme. Für diesen Wirkstoff sind verschiedene Verabreichungsintervalle vorgesehen, deren genaue Einhaltung überwacht werden muss, z.B. einmal täglich bei Patienten zur Verhinderung des Bluthochdrucks, zweimal täglich zur Verminderung des Reinfarktrisikos und bei Anzeichen von Angina Pectoris sowie dreimal täglich zur Verhinderung von Herzrhythmusstörungen.

Das erfindungsgemässe orale osmotische System erlaubt eine einmal tägliche Verabreichung für alle genannten Indikationen, wobei von der Einheitsdosis von ca. 60 bis 500 mg Metoprolol ca. 50 bis 90 % der Gesamtmenge mit der kontinuierlichen Abgabegeschwindigkeit von ca. 5 bis 12 % pro Stunde abgegeben werden.

Der Gegenstand der vorliegenden Erfindung wird im folgenden beschrieben:

Das erfindungsgemässe orale osmotische System enthält ein mässig wasserlösliches und pharmazeutisch annehmbares Metoprololsalz und eignet sich für die Abgabe einer festgelegten Wirkstoffmenge von ca. 50 bis 500 mg Metoprolol, wobei ca. 60 bis 90 % des Metoprololsalzes mit einer weitgehend kontinuierlichen Geschwindigkeit von ca. 5 bis 12 % Gewichtsprozent bezogen auf das Gesamtgewicht der Formulierung abgegeben werden.

Das in der erfindungsgemässen Darreichungsform verwendete pharmazeutisch annehmbare Metoprololsalz ist nur mässig wasserlöslich und löst sich in wässrigem Milieu nach oraler Einnahme langsam auf, indem im gastrointestinalen Trakt Flüssigkeit durch Diffusion die semipermeable Wand des Systems durchdringt, den Kern des Systems erreicht und dort kontinuierlich eine osmotisch aktive Lösung des gelösten Metoprololsalzes erzeugt. Die mit gelöstem Metoprololsalz angereicherte Lösung erzeugt ein osmotisches Druckgefälle gegen die wässrige Flüssigkeit im Gastrointestinaltrakt. Die Wirkstofflösung wird durch eine oder mehrere Passagen in der Umhüllung des therapeutischen Systems, welche zwischen dem Kern und der umgebenden Körperflüssigkeit eine Verbindung herstellt, abgegeben, so dass man eine verzögerte und kontrollierte Abgabeleistung vorzugsweise mit konstanter Abgabegeschwindigkeit erhält (Nullte Ordnung). Die Aufnahmegeschwindigkeit von Flüssigkeit durch die semipermeable Umhüllung wird durch die kontinuierliche Auflösungsgeschwindigkeit im Kern des Systems gesteuert. Man verwendet als Metoprololsalz nur ein solches Salz mit mässiger Wasserlöslichkeit, welches eine langsame und kontinuierliche Abgabe des Wirkstoffs aus dem Kern des Systems gewährleistet.

Vorzugsweise besitzt ein pharmazeutisch annehmbares Salz von Metoprolol, welches sich für die Verwendung in oralen osmotischen Systemen eignet, eine Löslichkeit von ca. 0,1 bis 0,6 g pro $cm^3$ bei ca. 37°C. Geeignete Metoprololsalze sind deren Niederalkanoate, insbesondere die Mono- oder die Dimetoprololsalze von Niederalkylendicarbonsäuren, z.B. die Fumarat- oder Maleatmono- oder -di-Salze von Metoprolol. Besonders bevorzugt ist das Monometoprololfumarat (1:1).

Die äussere Hülle a) aus einem für Wasser durchlässigen und für die Komponenten des wirkstoffhaltigen Kerns undurchlässigen semipermeablen Material hat film- oder schichtbildende Eigenschaften und ist sowohl gegenüber den Bestandteilen des wirkstoffhaltigen Kerns als auch der umgebenden Körperflüssigkeit inert. Das Material muss sich für die Verwendung in der Pharmazie eignen, für die umgebende Körperflüssigkeit durchlässig und für das Metoprololsalz im wesentlichen undurchlässig sein. Das Material ist in der umgebenden Körperflüssigkeit schwerlöslich oder soweit löslich, dass dieses erst gegen Ende des Durchtritts durch den Gastrointestinaltrakt gelöst ist. Für die Bildung der äusseren Hülle geeignete Materialien sind. z.B. für die Herstellung von osmotischen Membranen oder Umkehrosmosemembranen bekannt und z.T. kommerziell erhältlich, z.B. Celluloseacetat, Cellulosetriacetat, Agaracetat, Amylosetriacetat, beta-Glucanacetat, beta-Glucantriacetat, Celluloseacetatäthylcarbamat, Celluloseacetatphthalat, Cellulo-seacetatmethylcarbamat, Celluloseacetatsuccinat, Celluloseacetatdimethylaminoacetat, Celluloseacetatäthyl-carbonat, Celluloseacetatmethylsulfonat, Celluloseacetatbutylsulfonat, Celluloseacetatpropionat, Polyvinylmethyläther-Polymere, Celluloseacetatoctat, Celluloseacetatlaurat, Methylcellulose, Celluloseacetatp-toluolsulfonat, Aethylcellulose, Triacetat von Johanniskernmehl, Celluloseacetat mit acetylierter Hydroxy-äthylcellulose, Hydroxyliertes Aethylenvinylacetat, osmotische Membranen aus polymeren Epoxyden, Alkenylenoxid-Alkylglycidyl Aethern, Polyurethanen, Polyglycolsäure, und übliche Polycation-Polyanion Membranen.

Generall haben geeignete Membranen für Flüssigkeiten eine Permeabilität von ca. 0,01 bis 10 ml/cm² pro Stunde oder Tag bei Atmosphärendruck oder erhöhtem Druck gegen eine gesättigte Lösung bei ca. 30°C und sind gleichzeitig gegenüber Metoprololsalzen undurchlässig.

Besonders bevorzugte Membranmaterialien sind Polyurethane, Methylcellulose, Aethylcellulose oder Celluloseacetatbutyrat. Besonders bevorzugt ist vor allem Celluloseacetat.

Die genannten semipermeablen Membranmaterialien sind bekannt und mit ihren Eigenschaften und Verwendungsmöglichkeiten in der U.S. Patentschrift 3,916,899 beschrieben.

Für die Herstellung von festen Darreichungsformen übliche Schmiermittel sind z.B. Silica, Talk oder hochmolekulares Polyäthylenglycol. Besonders bevorzugt ist Magnesiumstearat, insbesondere in einem Mengenverhältnis von ca. 1 bis 5 %, vorzugsweise ca. 2 bis 4 %, bezogen auf das Gesamtgewicht des Kerns.

Das im Kern b) vorhandene Poly-N-vinylpyrrolidon ist wasserlöslich oder quellfähig und hat eine mittlere Molmasse von ca. 10'000 bis 700'000, bevorzugt 10'000-100'000. Synonyme Bezeichnungen sind Polyvidone, Polyvinylpyrrolidon, Povidone, PVP, KOLLIDON (BASF) oder PLASDON (GAF Corp.). Poly-N-vinylpyrrolidon ist mit einem Gehalt von 7,5-15 Gew-% bezogen auf das Gesamtgewicht des Kerns b) im Kern des oralen osmotischen Systems vorhanden.

Vorzugsweise ist Poly-N-vinylpyrrolidon mit einem Gehalt von 8,5 bis 13,0 % im Kern vorhanden. Das Poly-N-vinylpyrrolidon wird in pharmazeutischer Qualität verwendet. Auf die Reinheitsvorschriften z.B. gemäss U.S. Pharmacopoeia (USP), British Pharmacopoeia (BP) oder Deutsches Arzneibuch 7 wird verwiesen. Besonders bevorzugt ist Poly-N-vinylpyrrolidon mit einem K-Wert von 30, was einer mittleren Molmasse von ca. 40'000 entspricht.

Die Menge des im Kern b) vorhandenen Metoprololsalzes ist variierbar und entspricht der für Wirkstoff Metoprolol zulässigen Höchst- und Mindestdosierung. Es können im therapeutischen System ca. 50-500 mg Metoprolol vorhanden sein, vorzugsweise 60-200 mg Wirkstoff.

Der Kern b) liegt vorzugsweise als tablettenförmige Formulierung vor, welche mit dem Membranmaterial der Hülle a) überzogen ist. Die Herstellung des Kerns kann beispielsweise erfolgen, indem das mässig wasserlösliche Metoprololsalz mit dem Poly-N-vinylpyrrolidon direkt trocken verpresst oder als Granulat verpresst wird. Die Herstellung des letzteren erfolgt z.B. durch Vermischen und Vortrocknen einer wässrig äthanolischen Suspension der Komponenten und Zerkleinerung und Trocknen des Rückstandes oder durch Auflösen oder Suspendieren des Metoprololsalzes in einer wässrigen äthanolischen Lösung von Poly-N-vinylpyrrolidon, Vortrocknen, Zerkleinern, Entfernen des restlichen Lösungsmittels, Mahlen des Granulats auf einheitliche Kerngrösse und Verpressen des Granulats in Gegenwart eines Gleitmittels wie Magnesiumstearat zu tablettenförmigen Kernen.

Durch Verwendung von Poly-N-vinylpyrrolidon als Hilfsstoff in der angegebenen Menge von 7,5-15 %, vorzugsweise 8,5-13 %, lassen sich tablettenförmige Kerne geeigneter Härte herstellen, deren Härtegrade ca. 8-25 SCU (Strong Cobb Units, Strong Cobb hardness tester, pg. 1608 Remington's Pharmaceutical Sciences, March 1985), insbesondere 8-20 SCU, und Bruchfestigkeit, gemessen in prozentualen Anteilen an zerbrochenen Tablettenkernen, nicht grösser als 2,5 %, insbesondere nicht grösser als 1% ist.

Dis äussere Hülle a) kann auf den den Wirkstoff enthaltenden Kern durch Aufgiessen, Formen, Sprühen oder Eintauchen der Kapsel in das die semipermeable Hülle bildende Material aufgebracht werden. Ein anderes Verfahren, das zum Aufbringen der Hülle angewandt werden kann, besteht im Luftverwirbeln (air suspension procedure). Dieses Verfahren besteht darin, dass man die Massen (Kapseln bzw. Kapselkerne) in einem Luftstrom und einem die Hülle bildenden Mittel suspendiert und stürzt, bis die Hülle den Kern umgibt und überzieht.

Das Luftverwirbelungs-Verfahren ist in der U.S. Patentschrift 2 799 241 sowie im J.Am. Pharm. Assoc., Bd. 48, S. 451 - 459, 1979, und im Band 49, S. 82 bis 84, 1980, beschrieben. Andere bevorzugte Standardverfahren sind beispielsweise das Kessellackierungsverfahren (spray pan), welches in Remington's Pharmaceutical Sciences, 14th Edition, auf den Seiten 1686 - 1687 beschrieben ist. Bei Verwendung von Celluloseacetat als membranbildendem semipermeablem Material hat die Hülle a) einen Anteil von ca. 4-30 % (Gew.), vorzugsweise 10-20 %, an Gesamtgewicht der Formulierung.

Der Begriff "Passage durch die Hülle a) für den Transport der im Kern enthaltenen Bestandteile in die imgebende wässrige Körperflüssigkeit" umfasst Vorrichtungen sowie Methoden, die geeignet sind, die Wirkstoffzubereitung aus dem Kern des therapeutischen Systems freizusetzen. Der Begriff umfasst Durchgänge, Oeffnungen, Bohrungen, Löcher u.ä. durch die als semipermeable Membran wirkende Hülle a), welche zwischen der Oberfläche der Hülle und dem Kern eine Verbindung herstellen. Die Passage kann durch mechanisches Bohren oder Laserbohren oder durch Zersetzen eines abbaubaren Bestandteils, z.B. eines Gelatinestopfens, unter Bildung einer Passage in der Hülle des therapeutischen Systems hergestellt werden. Bei einer Ausführungsform kann sich die Passage als Reaktion auf den hydrostatischen Druck bilden, welcher auf das therapeutische System einwirkt. Bei einer anderen Ausführungsform können zwei oder mehrere Durchgänge hergestellt werden, die sich an einer beliebigen Stelle des Systems befinden. Die Passage kann auch durch mechanisches Aufbrechen der Schichten während der Anwendung des Systems entstehen. Die Passage hat einen minimalen Durchmesser, der von der Korngrösse der Wirkstoffkristalle abhängig ist. Der Durchmesser der Passage muss grösser als die durchschnittliche Länge der Wirkstoffkristalle sein. Der maximale Durchmesser ist ebenfalls annähernd festgelegt. Dieser darf nur so gross sein, dass das Eindringen von wässriger Körperflüssigkeit durch Konvektion in das therapeutische System vermieden wird. Eine genaue Beschreibung der Herstellung der Passage und der maximalen und minimalen Dimensionen davon ist in den U.S. Patentschriften 3 485 770 und 3 916 899 und den dazugehörigen Zeichnungen enthalten. Die Grösse der Oeffnung beträgt in einer bevorzugten Ausführungsform ca. 0,1-0,8 mm.

4

Das erfindungsgemässe therapeutische System kann unterschiedlich geformt sein und beispielsweise rund, oval, oblong, zylindrisch u.ä. sein sowie verschiedene Grössen in Abhängigkeit von der Füllmenge haben. Das therapeutische System kann ausserdem transparent, farblos oder gefärbt und gegebenenfalls beschriftet sein, um diesem Produkt ein individuelles Aussehen bzw. sofortige Erkennbarkeit zu verleihen.

Die Passage in der semipermeablen Hülle kann anschliessend durch Bohren mechanisch oder mit Laser erzeugt werden. Die folgenden Beispiele illustrieren die Erfindung.

Beispiel 1:

95,37 Gewichtsteile Metoprololfumarat (1:1-Salz) werden mit 8,10 Gewichtsteilen Povidon (PLASDON K-30 GAF) vermischt, zu einem Pulver gemahlen und mit einer Aethanol/Wasser-Mischung (70:30) granuliert. Das Granulat wird getrocknet, gesiebt und mit 1,53 Gewichtsteilen Magnesiumstearat vermischt. Das Granulatgemisch wird anschliessend zu tablettenförmigen Kernen verpresst, welche bei einem Gesamtgewicht von 105 mg einen Anteil von 7,7 Gew-% Povidon und 1,5 Gew-% Magnesiumstearat enthalten, wobei der Rest den Wirkstoffanteil in Form des Metoprololsalzes ausmacht. Die tablettenförmigen Kerne haben folgende mechanischen Eigenschaften:

| | |
|---|---|
| Härte (Strong Gobb Unit, SCU): | 9-11 |
| Bruchfestigkeit (%-Anteil zerbrochene Tablettenkerne): | 0,2 |
| Zerfallszeit: | 9 Min. |

Beispiel 2:

Vergleich der Bruchfestigkeit von Tablettenkernen mit geringerem Anteil an Poly-N-vinylpyrrolidon. Ansonsten Herstellung analog Beispiel 1.

| | Bestandteile in Gewichtsteilen: | Gewichtsprozente |
|---|---|---|
| Metoprololfumarat (1:1) | 95,37 | 93,5 |
| Povidon USP | 5,10 | 5 |
| Magnesiumstaearat NF | 1,53 | 1,5 |

Härte : 7-9
Bruchfestigkeit : 7,2 (beschichtete Tablettenkerne)
Zerfallszeit : 8 Minuten

Beispiele 3-6:

Analog den Verfahren gemäss Beispiel 1 werden folgende tablettenförmige Kerne hergestellt:

| Beispiel | 3 | 4 | 5 | 6 |
|---|---|---|---|---|
| Metoprololfumarat (1:1) | 95.37 | 95.37 | 95.37 | 95.37 |
| Povidone, USP | 10.10 | 13.10 | 8.10 | 11.10 |
| Magnesiumstearat NF | 1.53 | 1.53 | 2.53 | 2.53 |
| % Povidone | 9.4 | 12.0 | 7.6 | 10.2 |
| % Magnesiumstearat | 1.4 | 1.4 | 2.4 | 2.3 |
| Härte (SCU) | 14-15 | 11-18 | 9-11 | 8-11 |
| Bruchfestigkeit | 0.3 | 0.2 | 0.2 | 0.2 |
| Zerfallszeit (Min.) | 7 | 8 | 18 | 15 |

Beispiel 7:

Gemäss dem in Beispiel 1 beschriebenen Verfahren werden tablettenförmige Kerne hergestellt, welche pro Kern 95 mg Metoprololfumarat, 2,9 mg Magnesiumstearat NF und 11,10 mg Povidone USP (PLASDON K-30) enthalten und einen Härtegrad von 8-11 (SCU), eine Bruchfestigkeit von 0,2 % und eine Zerfallszeit von 15 Minuten besitzen. Die tablettenförmigen Kerne werden nach Wursters Luftverwirbelungsverfahren mit einer Lösung von Celluloseacetat in einem Methanol/Methylenchlorid Gemisch überzogen, dass jede Tablette mit 16 mg Celluloseacetat beschichtet ist. Man bohrt in diese Schicht eine Oeffnung von ca. 0,5 mm Durchmesser.

Beispiel 8:

Beschichtete Iablettenkerne werden analog Beispiel 7 hergestellt und mit einer Bohrung versehen. Das so erhältliche therapeutische System hat pro Stück im Kern folgende Zusammensetzung: Metoprololfumarat: 190,74 mg, Povidone USP (PLASDON K-30): 22,20 mg und Magnesiumstearat: 5,06 mg. Der Kern hat folgende mechanische Eigenschaften: Härtegrad (SCU): 11-16; Bruchfestigkeit: 0,2 %; Zerfallszeit: 27 Min. Der Kern ist mit einer Beschichtung aus Celluloseacetat (27 mg) überzogen, worin eine Oeffnung von 0,5 mm Durchmesser angebracht ist. Analog den in U.S. Pharmacopoeia SS. 1243-1244 für die Messung von Auflösungsvorgängen von festen Darreichungsformen beschriebenen Verfahren wird das Abgabeverhalten eines therapeutischen Systems bei 37°C in artifiziellem Magensaft (ohne Enzyme) bei 100 Umdrehungen pro Minute Rührgeschwindigkeit untersucht und folgende Werte ermittelt:

| Zeit [h] | Durchschnittliche Abgabegeschwindigkeit [mg/h] | Durchschnittliche abgegebene Gesamtmenge [%] |
|---|---|---|
| 0 - 2 | 17.8 ± 2.7 | 18.7 |
| 2 - 4 | 21.4 ± 1.6 | 41.2 |
| 4 - 6 | 19.0 ± 0.9 | 61.2 |
| 6 - 8 | 17.0 ± 1.6 | 79.0 |
| 8 - 10 | 8.3 ± 1.4 | 87.7 |
| 10 - 12 | 4.5 ± 0.7 | 92.5 |

**Patentansprüche**

**Patentansprüche für folgende Vertragsstaaten : AT, BE, CH, DE, FR, GB, IT, LI, LU, NL, SE**

**1.** Orales osmotisches System für die Verabreichung von Metoprolol bestehend aus:
a) einer äusseren Hülle aus einem für Wasser durchlässigen und für die Komponenten des wirkstoffhaltigen Kerns undurchlässigen semipermeablem Material,
b) einem Kern mit einem zur Erzeugung von osmotischem Druck ausreichend wasserlöslichen Gemisch aus 7,5 bis 15 Gew-% Poly-N-vinylpyrrolidon, ca. 5 Gew-% eines bei der Herstellung von festen Darreichungsformen üblichen Gleit- oder Schmiermittels und 80 bis 92,5 % eines mässig wasserlöslichen und pharmazeutisch annehmbaren Metoprololsalzes und
c) einer Passage durch die Hülle a) für den Transport der im Kern enthaltenen Bestandteile in die umgebende wässrige Körperflüssigkeit.

**2.** Orales osmotisches System gemäss Anspruch 1, dadurch gekennzeichnet, dass der Kern b) 8,5-13 % Poly-N-vinylpyrrolidon enthält.

**3.** Orales osmotisches System gemäss Anspruch 1, dadurch gekennzeichnet, dass der Kern b) 8,5-13 % Poly-N-vinlypyrrolidon mit einer mittleren Molmasse von ca. 40,000 enthält.

**4.** Orales osmotisches System gemäss Anspruch 1, dadurch gekennzeichnet, dass der Kern b) als mässig wasserlösliches und pharmazeutisch annehmbares Metoprololsalz das Fumaratsalz von Metoprolol (1:1) enthält.

**5.** Orales osmotisches System gemäss Anspruch 1, dadurch gekennzeichnet, dass die Hülle a) aus Celluloseacetat besteht.

**6.** Orales osmotisches System gemäss Anspruch 1, dadurch gekennzeichnet, dass die in der Hülle b) enthaltene Menge an Metoprololsalz 60 bis 200 mg freiem Metoprolol entspricht.

**7.** Orales osmotisches System gemäss Anspruch 1, dadurch gekennzeichnet, dass man Magnesiumstearat als Gleit- oder Schmiermittel verwendet.

**8.** Orales osmotisches System gemäss Anspruch 1 zur Anwendung in einem therapeutischen Verfahren, worin die Verwendung von Beta-Adrenalinrezeptorblockern indiziert ist.

**9.** Verfahren zur Herstellung eines oralen osmotischen Systems gemäss Anspruch 1, dadurch gekennzeichnet, das man den Kern b) mittels üblicher Tablettierungsverfahren durch Direktverpressung oder Granulierung herstellt, diesen Kern mit der äusseren Hülle a) überzieht und in dieser Hülle die Passage c) appliziert.

**Patentansprüche für folgende Vertragsstaaten : ES, GR**

**1.** Verfahren zur Herstellung eines oralen osmotisches System für die Verabreichung von Metoprolol bestehend aus:
a) einer äusseren Hülle aus einem für Wasser durchlässigen und für die Komponenten des wirkstoffhaltigen Kerns undurchlässigen semipermeablem Material,
b) einem Kern mit einem zur Erzeugung von osmotischem Druck ausreichend wasserlöslichen Gemisch aus 7,5 bis 15 Gew-% Poly-N-vinylpyrrolidon, ca. 5 Gew-% eines bei der Herstellung von festen Darreichungsformen üblichen Gleit- oder Schmiermittels und 80 bis 92,5 % eines mässig wasserlöslichen und pharmazeutisch annehmbaren Metoprololsalzes und
c) einer Passage durch die Hülle a) für den Transport der im Kern enthaltenen Bestandteile in die umgebende wässrige Körperflüssigkeit, dadurch gekennzeichnet, das man den Kern b) mittels üblicher Tablettierungsverfahren durch Direktverpressung oder Granulierung herstellt, diesen Kern mit der äusseren Hülle a) überzieht und in dieser Hülle die Passage c) appliziert.

**2.** Verfahren zur Herstellung eines oralen osmotisches System gemäss Anspruch 1, dadurch gekennzeichnet, dass der Kern b) mit einem Zusatz von 8,5-13 % Poly-N-vinylpyrrolidon formuliert..

**3.** Verfahren zur Herstellung eines oralen osmotisches System gemäss Anspruch 1, dadurch gekennzeichnet, dass der Kern b) mit einem Zusatz von 8,5-13 % Poly-N-vinlypyrrolidon mit einer mittleren Molmasse von ca. 40,000 formuliert.

**4.** Verfahren zur Herstellung eines oralen osmotisches System gemäss Anspruch 1, dadurch gekennzeichnet, dass der Kern b) als mässig wasserlösliches und pharmazeutisch annehmbares Metoprololsalz das Fumaratsalz von Metoprolol (1:1) enthält.

**5.** Verfahren zur Herstellung eines oralen osmotisches System gemäss Anspruch 1, dadurch gekennzeichnet, dass man den Kern b) mit einer Hülle a) aus Celluloseacetat überzieht.

**6.** Verfahren zur Herstellung eines oralen osmotischen Systems gemäss Anspruch 1, dadurch gekennzeichnet, dass die in der Hülle b) enthaltene Menge an Metoprololsalz 60 bis 200 mg freiem Metoprolol entspricht.

**7.** Verfahren zur Herstellung eines oralen osmotischen Systems gemäss Anspruch 1, dadurch gekennzeichnet, dass man Magnesiumstearat als Gleit- oder Schmiermittel verwendet.

**Claims**

**Claims for the following Contracting States : AT, BE, CH, DE, FR, GB, IT, LI, LU, NL, SE**

1. An oral osmotic system for the administration of metoprolol, comprising:
   a) an outer casing made of a semi-permeable material that is permeable to water and impermeable to the components of the core containing active ingredient;
   b) a core that comprises a mixture, which is sufficiently water-soluble to produce osmotic pressure, of 7.5 to 15 percent by weight poly-N-vinylpyrrolidone, about 5 percent by weight of a glidant or lubricant customary in the preparation of solid dosage forms, and 80 to 92.5 percent by weight of a moderately water-soluble, pharmaceutically acceptable metoprolol salt; and
   c) a passageway through the casing a) for transporting the constituents contained in the core into the surrounding aqueous body fluid.

2. An oral osmotic system according to claim 1, wherein the core b) comprises 8.5-13 % poly-N-vinylpyrrolidone.

3. An oral osmotic system according to claim 1, wherein the core b) comprises 8.5-13 % poly-N-vinylpyrrolidone having an average molecular weight of about 40 000.

4. An oral osmotic system according to claim 1, wherein the core b) comprises the fumarate salt of metoprolol (1:1) as the moderately water-soluble, pharmaceutically acceptable metoprolol salt.

5. An oral osmotic system according to claim 1, wherein the casing a) consists of cellulose acetate.

6. An oral osmotic system according to claim 1, wherein the amount of metoprolol salt contained in the casing b) corresponds to 60 to 200 mg of free metoprolol.

7. An oral osmotic system according to claim 1, wherein the glidant or lubricant is magnesium stearate.

8. An oral osmotic system according to claim 1 for use in a therapeutic method wherein the use of beta-adreno-receptor blocking agents is indicated.

9. A process for the preparation of an oral osmotic system according to claim 1, which comprises producing the core b) by means of customary tabletting processes by direct compressing or granulating, covering the core with the outer casing a) and forming the passageway c) in the casing.

**Claims for the following Contracting States : ES, GR**

1. A process for the preparation of an oral osmotic system for the administration of metoprolol, comprising:
   a) an outer casing made of a semi-permeable material that is permeable to water and impermeable to the components of the core containing active ingredient;
   b) a core that comprises a mixture, which is sufficiently water-soluble to produce osmotic pressure, of 7.5 to 15 percent by weight poly-N-vinylpyrrolidone, about 5 percent by weight of a glidant or lubricant customary in the preparation of solid dosage forms, and 80 to 92.5 percent by weight of a moderately water-soluble, pharmaceutically acceptable metoprolol salt; and
   c) a passageway through the casing a) for transporting the constituents contained in the core into the surrounding aqueous body fluid,
   which process comprises producing the core b) by means of customary tabletting processes by direct compressing or granulating, covering the core with the outer casing a) and forming the passageway c) in the casing.

2. A process for the preparation of an oral osmotic system according to claim 1, which comprises formulating the core b) with an addition of 8.5-13 % poly-N-vinylpyrrolidone.

**3.** A process for the preparation of an oral osmotic system according to claim 1, which comprises formulating the core b) with an addition of 8.5-13 % poly-N-vinylpyrrolidone having an average molecular weight of about 40 000.

**4.** A process for the preparation of an oral osmotic system according to claim 1, wherein the fumarate salt of metoprolol (1:1) is contained as the moderately water-soluble, pharmaceutically acceptable metoprolol salt for the core b).

**5.** A process for the preparation of an oral osmotic system according to claim 1, wherein the core b) is covered with a casing a) consisting of cellulose acetate.

**6.** A process for the preparation of an oral osmotic system according to claim 1, wherein the amount of metoprolol salt contained in the casing b) corresponds to 60 to 200 mg of free metoprolol.

**7.** A process for the preparation of an oral osmotic system according to claim 1, wherein the glidant or lubricant is magnesium stearate.

**Revendications**

**Revendications pour les Etates contractants suivants : AT, BE, CH, DE, FR, GB, IT, LI, LU, NL, SE**

**1.** Système osmotique oral pour l'administration du Metoprolol, consistant en :
a) une enveloppe extérieure consistant en une matière semi-perméable, perméable à l'eau et imperméable pour les composants du noyau contenant la substance active,
b) un noyau avec un mélange, suffisamment soluble dans l'eau pour produire une pression osmotique, de 7,5 à 15% en poids de poly-N-vinylpyrrolidone, d'environ 5% en poids d'un agent lubrifiant usuel pour la préparation des formes d'administration solides et 15 à 92,5% en poids d'un sel de Metoprolol acceptable pour l'usage pharmaceutique et à solubilité limitée dans l'eau, et
c) un passage au travers de l'enveloppe a) pour le transport des composants contenus dans le noyau vers le liquide physiologique aqueux environnant.

**2.** Système osmotique oral selon revendication 1, caractérisé en ce que le noyau b) contient de 8,5 à 13% de poly-N-vinylpyrrolidone.

**3.** Système osmotique oral selon revendication 1, caractérisé en ce que le noyau b) contient de 8,5 à 13% de poly-N-vinylpyrrolidone à une masse moléculaire moyenne d'environ 40 000.

**4.** Système osmotique oral selon revendication 1, caractérisé en ce que le noyau b) contient en tant que sel du Metoprolol acceptable pour l'usage pharmaceutique et à solubilité limitée dans l'eau, le fumarate (1:1) du Metoprolol.

**5.** Système osmotique oral selon revendication 1, caractérisé en ce que l'enveloppe a) consiste en acétate de cellulose.

**6.** Système osmotique oral selon revendication 1, caractérisé en ce que la quantité de sel de Metoprolol contenue dans l'enveloppe b) correspond à 60 à 200 mg de Metoprolol libre.

**7.** Système osmotique oral selon revendication 1, caractérisé en ce que l'on utilise le stéarate de magnésium en tant qu'agent lubrifiant.

**8.** Système osmotique oral selon revendication 1, pour l'utilisation dans un procédé thérapeutique dans lequel l'utilisation des bêta-bloquants des récepteurs de l'adrénaline est indiquée.

**9.** Procédé de préparation d'un système osmotique oral selon revendication 1, caractérisé en ce que l'on prépare le noyau b) par des procédés classiques de mise en comprimés, par compression directe ou mise en granulés, on revêt ce noyau de l'enveloppe extérieure a) et on pratique dans cette enveloppe le passage c).

**Revendications pour les Etats contractants suivants : ES, GR**

1. Procédé de préparation d'un système osmotique oral pour l'administration du Metoprolol, consistant en :

   a) une enveloppe extérieure en une matière semiperméable, perméable pour l'eau et imperméable pour les composants du noyau contenant la substance active,

   b) un noyau avec un mélange, suffisamment soluble dans l'eau pour produire une pression osmotique, de 7,5 à 15% en poids de poly-N-vinylpyrrolidone, environ 5% en poids d'un agent lubrifiant usuel pour la préparation des formes d'administration solides et 80 à 92,5% d'un sel de Metoprolol acceptable pour l'usage pharmaceutique et à solubilité limitée dans l'eau, et

   c) un passage au travers de l'enveloppe a) pour le transport des composants contenus dans le noyau vers le liquide physiologique aqueux environnant, caractérisé en ce que l'on prépare le noyau b) par des procédés usuels de mise en comprimés, par compression directe ou par mise en granulés, on revêt ce noyau de l'enveloppe extérieure a) et on pratique dans cette enveloppe le passage c).

2. Procédé de préparation d'un système osmotique oral selon revendication 1, caractérisé en ce que le noyau b) est préparé avec adjonction de 8,5 à 13% de poly-N-vinylpyrrolidone.

3. Procédé de préparation d'un système osmotique oral selon revendication 1, caractérisé en ce que l'on prépare le noyau b) avec adjonction de 8,5 à 13% d'une poly-N-vinylpyrrolidone à une masse moléculaire moyenne d'environ 40 000.

4. Procédé de préparation d'un système osmotique oral selon revendication 1, caractérisé en ce que le noyau b) contient en tant que sel de Metoprolol acceptable pour l'usage pharmaceutique et à solubilité limitée dans l'eau, le fumarate (1:1) du Metoprolol.

5. Procédé de préparation d'un système osmotique oral selon revendication 1, caractérisé en ce que l'on revêt le noyau b) d'une enveloppe a) en acétate de cellulose.

6. Procédé de préparation d'un système osmotique oral selon revendication 1, caractérisé en ce que la quantité de sel de Metoprolol contenue dans l'enveloppe b) correspond à 60 à 200 mg de Metoprolol libre.

7. Procédé de préparation d'un système osmotique oral selon revendication 1, caractérisé en ce que l'on utilise le stéarate de magnésium en tant qu'agent lubrifiant.